# EUROPEAN PATENT APPLICATION

(11) **EP 1 142 584 A1**
(43) Date of publication of application: **10.10.2001**
(21) Application number: 00900169.4
(22) Date of filing: 11.01.2000
(51) Int. Cl.: A61K 45/00, A61P 13/02, A61K 31/44, A61K 31/445, A61K 31/40, A61K 31/428

(54) **COMPOSITIONS FOR TREATING FREQUENT URINATION AND URINARY INCONTINENCE**

(30) Priority: 12.01.1999 JP 555699
(71) Applicant: Takeda Chemical Industries, Ltd., Osaka-shi, Osaka 541-0045 (JP)
(72) Inventor: HASHIMOTO, Tadatoshi, Ibaraki-shi Osaka 567-0828 (JP); KAMO, Izumi, Amagasaki-shi Hyogo 661-0047 (JP)
(74) Representative: von Kreisler, Alek, Dipl.-Chem.
(86) International application number: JP0000074
(87) International publication number: WO0041728

(57) **Abstract**

A GABA uptake inhibitor is useful in composition for treating urinary frequency and urinary incontinence.

## Description

### Technical Field

The present invention relates to a composition for preventing or treating urinary frequency and urinary incontinence which comprises a GABA uptake inhibitor.

### Background Art

γ-Amino butyric acid (GABA) is an inhibitory neurotransmitter which is present in cerebellum, dorsal horn of spinal cord, substantia nigra and hippocampus of a mammal in a large amount, and is biosynthesized mainly from glutamic acid by undergoing the action of glutamate decarboxylase mainly at a nerve ending of an inhibitory neuron in the central nervous system (CNS). GABA is released from a presynaptic part in response to the stimulation, a part thereof is uptaken again into a presynaptic site, and converted into succinic acid by GABA transaminase. A role of GABA in the nervous system is to increase the permeability of a postsynaptic membrane to Cl⁻ or K⁺, produce hyperpolarization and exert the inhibitory function (Seikagaku Jiten (Biochemistry Dictionary), 3rd. edition, 1998, Tokyo Kagaku Dojin).

A substance which inhibits this uptake of GABA is known to be useful as a drug for treating anxiety and epilepsy (USP 4,383,999) or an antitussive (WO97/43902).

In DK9800727, there is described that tiagabin which is a GABA uptake inhibitor, or a pharmacologically acceptable salt thereof is useful for treating incontinence. However, it is not described that tiagabin or a pharmacologically acceptable salt thereof has the excellent effect of treating urinary incontinence.

An object of the present invention is to provide a useful and excellent composition for preventing or treating urinary frequency and urinary incontinence.

### Disclosure of the Invention

In view of the above object, the present inventors studied intensively and, as a result, found that a GABA uptake inhibitor which is known as a drug for treating anxiety and epilepsy or antitussive is unexpectedly effective as a composition for preventing or treating urinary frequency or urinary incontinence, which resulted in the completion of the present invention.

That is, the present invention provides:
1. a composition for preventing or treating urinary frequency and urinary incontinence, which comprises a GABA uptake inhibitor;
2. the composition as defined in the above 1, wherein the GABA uptake inhibitor is:
   1) 1-(2-((diphenylmethylene)amino)oxy)ethyl)-1,2,5,6-tetrahydro-3-pyridinecarboxylic acid represented by the formula: 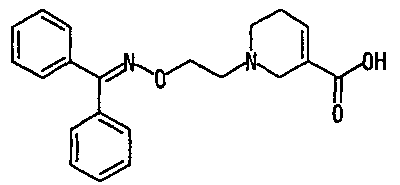 or a salt thereof,
   2) (R)-1-(4,4-diphenyl-3-butenyl)-3-piperidinecarboxylic acid represented by the formula: 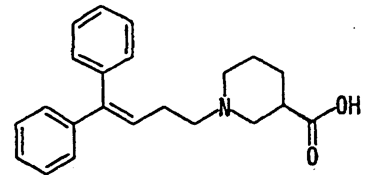 or a salt thereof,
   3) a compound represented by the formula: 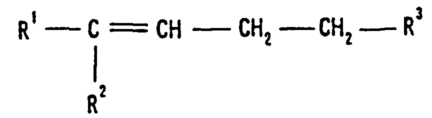 wherein R¹ and R² are the same or different, and each represents furanyl, thienyl, pyridyl or pyrrolyl, which may be substituted with 1 to 3 halogen or lower alkyl, and R³ represents 3-carboxypiperidin-1-yl, 3-carboxy-1,2,5,6-tetrahydropyrid-1-yl or 3-carboxymethylpyrrolidin-1-yl, or a salt thereof,
   4) 1,2,5,6-tetrahydronicotinic acid represented by the formula: 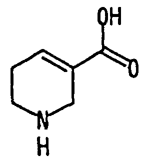 or a salt thereof,
   5) N-(4,4-diphenyl-3-butenyl)-3-pyrroridineacetic acid or a salt thereof,
   6) 3-piperidinecarboxylic acid or a salt thereof,
   7) 1,2,5,6-tetrahydro-3-pyridinecarboxylic acid or a salt thereof,
   8) N-(4,4-diphenyl-3-butenyl)-1,2,5,6-tetrahydro-3-pyridinecarboxylic acid or a salt thereof,
   9) cis-4-hydroxy-3-piperidinecarboxylic acid or a salt thereof,
   10) 6-(trifluoromethoxy)-2-benzothiazole-amine represented by the formula: 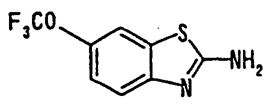 or a salt thereof, or
   11) a compound represented by the formula: 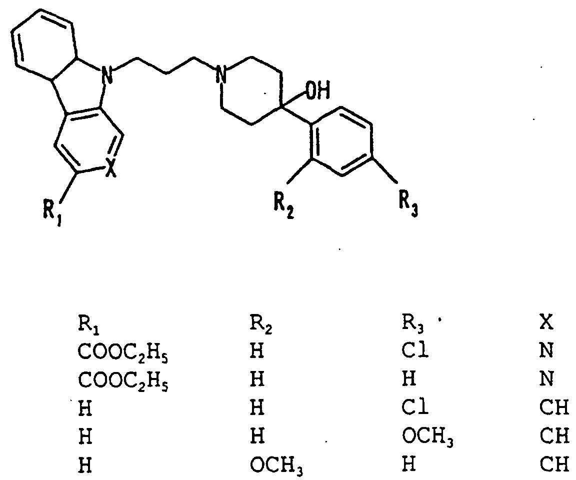 or a salt thereof;
3. the composition as defined in the above 1 which is that for preventing or treating urinary frequency;
4. the composition as defined in the above 3, wherein the GABA uptake inhibitor is:
   1) 1-(2-((diphenylmethylene)amino)oxy)ethyl)-1,2,5,6-tetrahydro-3-pyridinecarboxylic acid represented by the formula: 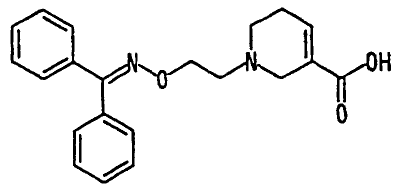 or a hydrochloride thereof,
   2) (R)-1-(4,4-diphenyl-3-butenyl)-3-piperidinecarboxylic acid represented by the formula: 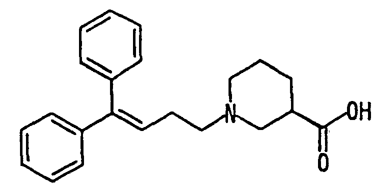 or a hydrochloride thereof, or
   3) (R)-1-[4,4-bis(3-methyl-2-thienyl)-3-butenyl]-3-piperidinecarboxylic acid represented by the formula: 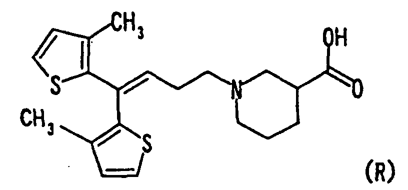 or a hydrochloride thereof;
5. the composition as defined in the above 1 which is that for preventing or treating urinary incontinence;
6. a composition for preventing or treating urinary incontinence, which comprises containing a GABA uptake inhibitor containing no sulfur atom;
7. the composition as defined in the above 5 or 6, wherein the GABA uptake inhibitor is:
   1) 1-(2-((diphenylmethylene)amino)oxy)ethyl)-1,2,5,6-tetrahydro-3-pyridinecarboxylic acid represented by the formula: 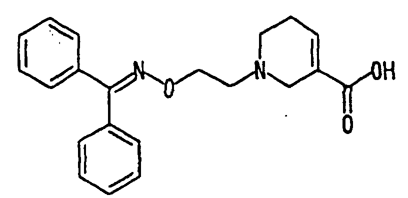 or a salt thereof,
   2) (R)-1-(4,4-diphenyl-3-butenyl)-3-piperidinecarboxylic acid represented by the formula: 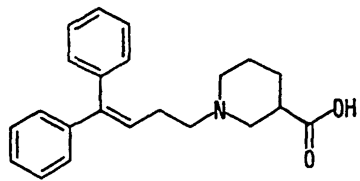 or a salt thereof,
   3) 1,2,5,6-tetrahydronicotinic acid represented by the formula: 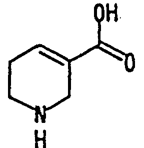 or a salt thereof,
   4) N-(4,4-diphenyl-3-butenyl)-3-pyrrolidineacetic acid or a salt thereof,
   5) 3-piperidinecarboxylic acid or a salt thereof,
   6) 1,2,5,6-tetrahydro-3-pyridinecarboxylic acid or a salt thereof,
   7) N-(4,4-diphenyl-3-butenyl)-1,2,5,6-tetrahydro-3-pyridinecarboxylic acid or a salt thereof,
   8) cis-4-hydroxy-3-piperidinecarboxylic acid or a salt thereof,
   9) 6-(trifluoromethoxy)-2-benzothiazole-amine represented by the formula: 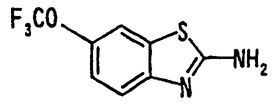 or a salt thereof, or
   10) a compound represented by the formula: 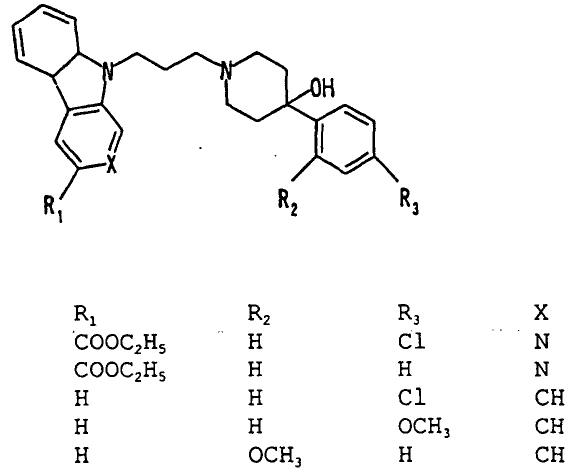 or a salt thereof;
8. the composition as defined in the above 5 or 6, wherein the GABA uptake inhibitor is:
   1) 1-(2-((diphenylmethylene)amino)oxy)ethyl)-1,2,5,6-tetrahydro-3-pyridinecarboxylic acid represented by the formula: 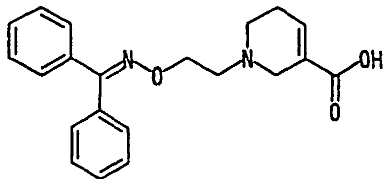 or a hydrochloride thereof, or
   2) (R)-1-(4,4-diphenyl-3-butenyl)-3-piperidinecarboxylic acid represented by the formula: 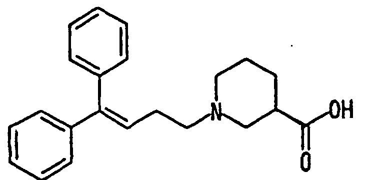 or a hydrochloride thereof;
9. the composition as defined in the above 5, wherein the GABA uptake inhibitor is a compound represented by the formula: 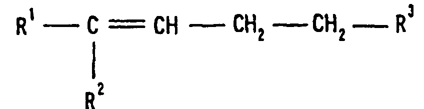 wherein R¹ and R² are the same or different, and each represents furanyl, thienyl, pyridyl or pyrrolyl, which may be substituted with 1 to 3 halogen or lower alkyl, and R³ represents 3-carboxy piperidin-1-yl, 3-carboxy-1,2,5,6-tetrahydropyrid-1-yl or 3-carboxymethylpyrrolidin-1-yl, or a salt thereof;
10. the composition as defined in the above 5, the GABA uptake inhibitor is (R)-1-[4,4-bis(3-methyl-2-thienyl)-3-butenyl]-3-piperidinecarboxylic acid represented by the formula: 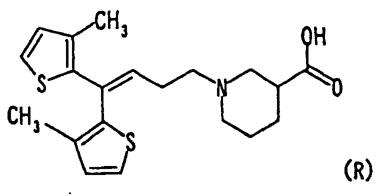 or a hydrochloride thereof;
11. the composition as defined in any one of the above 5 to 10, wherein urinary incontinence is urge urinary incontinence;
12. a method for preventing or treating urinary frequency and urinary incontinence, which comprises administering an effective amount of a GABA uptake inhibitor to a mammal; and
13. use of a GABA uptake inhibitor for preparing an agent for preventing or treating urinary frequency and urinary incontinence.

### Best Mode for Carrying Out the Invention

As used herein, "GABA uptake" means uptake of GABA by GABA nerve ending, glial cell or GABA cell surface transporting substance.

"GABA uptake inhibitor" may be any substance as long as it has the activity of inhibiting GABA uptake, such as a chemically Synthesized substance, a fermentationproduct and an genetic product, and may be the known substance or a novel substance which will be found out in the future.

The presence or the absence of the GABA uptake inhibiting activity can be measured by the method known per se, for example, a method described in USP 4,383,999, J. Med. Chem., 36, (1993) 1716-1725, Molecular & Cellular Biochemistry, 31, 105-121(1980) and the like, or a similar method.

As the GABA uptake inhibitor used in the present invention, the following compounds A-O, etc. are used. Inter alia, compound A, compound B and compound C (in particular, compound C-1), etc. are preferable, and compound C-1 is particularly preferable.
1) Compound A: 1-(2-((diphenylmethylene)amino)oxy)ethyl)-1,2,5,6-tetrahydro-3-pyridinecarboxylic acid (NO-711 or NNC-711) or a salt thereof (in particular, hydrochloride) (European Journal of Pharmacology, 223(1992) 189-198) 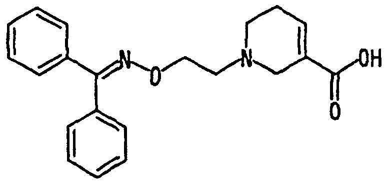 or a salt thereof (in particular, hydrochloride)
2) Compound B: (R)-1-(4,4-diphenyl-3-butenyl)-3-piperidinecarboxylic acid (SKF89976A) or a salt thereof (in particular, hydrochloride) (USP4,383,999 and J. Med. Chem., 1985, 28, 653-660) 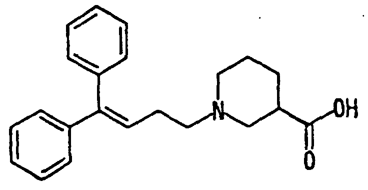 or a salt thereof (in particular, hydrochloride)
3) Compound C: a compound represented by the formula: 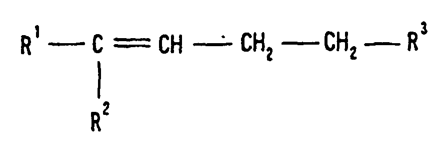 wherein R¹ and R² are the same or different, and each represents furanyl, thienyl, pyridyl or pyrrolyl, which may be substituted 1 to 3 halogen or lower alkyl, and R³ represents 3-carboxypiperidin-1-yl, 3-carboxy-1,2,5,6-tetrahydropyrid-1-yl or 3-carboxymethylpyrrolidin-1-yl, or a salt thereof (WO87/00171), more particularly,
   Compound C-1: (R)-1-[4,4-bis(3-methyl-2-thienyl)-3-butenyl]-3-piperidinecarboxylic acid (tiagabin) or a salt thereof (in particular, hydrochloride)(J. Med. Chem., 36, (1993) 1716-1725)) 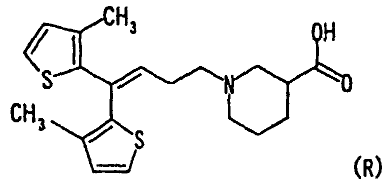
4) Compound D: 1,2,5,6-tetrahydronicotinic acid (gubacin) or a salt thereof (Molecular & Cellular Biochemistry, 31, 105-121 (1980)) 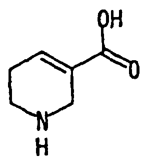
5) Compound E: N-(4,4-diphenyl-3-butenyl)-3-pyrrolidineacetic acid or a salt thereof (J. Med. Chem., 1985, 28, 653-660)
6) Compound F: 3-piperidinecarboxylic acid or a salt thereof (J. Med. Chem., 1985, 28, 653-660)
7) Compound G: 1,2,5,6-tetrahydro-3-pyridinecarboxylic acid or a salt thereof (J. Med. Chem., 1985, 28, 653-660)
8) Compound H: N-(4,4-diphenyl-3-butenyl)-1,2,5,6-tetrahydro-3-pyridinecarboxylic acid or a salt thereof (J. Med. Chem., 1985, 28, 653-660)
9) Compound I: cis-4-hydroxy-3-piperidinecarboxylic acid or a salt thereof (J. Med. Chem., 1985, 28, 653-660)
10) Compound J: 6-(trifluoromethoxy)-2-benzothiazole-amine (liruzole) or a salt thereof (USP4,370,338) 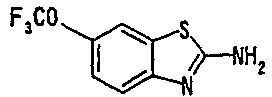
11) Compounds K to O or salts thereof (British Journal of Pharmacology, 1997, 120, 983-985)

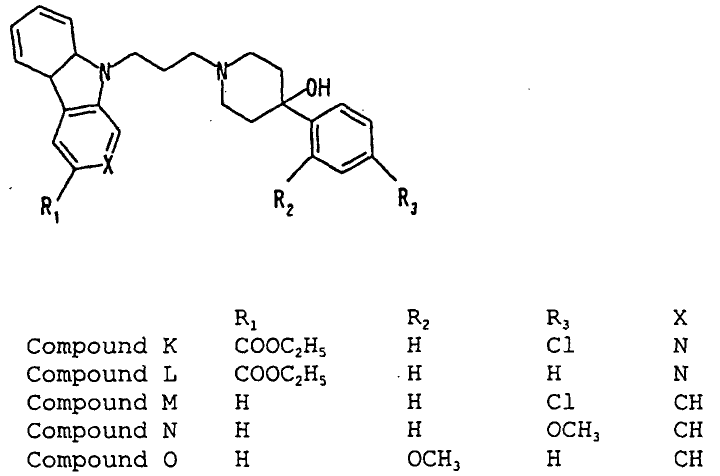

As a salt of these compounds, for example, salts with inorganic acids (such as hydrochloric acid, phosphoric acid, hydrobromic acid and sulfuric acid), or salts with organic acids (such as acetic acid, formic acid, propionic acid, fumaric acid, maleic acid, succinic acid, tartaric acid, citric acid, malic acid, oxalic acid, methanesulfonic acid and benzenesulfonic acid) are used. Further, when these compounds have an acidic group such as carboxylic acid as a substituent, they may form a salt with inorganic bases (such as alkali metal and alkaline earth metal such as sodium, potassium, calcium and magnesium, and ammonia) or organic bases (such as tri-C₁₋₃ alkylamine such as triethylamine).

The effects of a GABA uptake inhibitor of preventing or treating urinary frequency and urinary incontinence can be measured by a method described in, for example, Eur. J. Pharmacol., 103, 41-50 (1984), and Neuroscience, 54, 827-837 (1993) or a similar method. More particularly, the effects of a GABA uptake inhibitor of preventing or treating urinary frequency can be measured according to methods described in Test Examples 1 to 5 described below.

As used herein, urinary frequency indicates that there is an intention or function to suppress micturition but the number of micturition is larger as compared with a normal time, or the number of micturition is larger as compared with a normal person. More particularly, urinary frequency indicates that the number of micturition is about 1.5-fold or more the number of micturition at a normal time or that of a normal person and, in the case of severer conditions, about 2 to 3-fold or more.

As used herein, urinary incontinence indicates that an intention or function to suppress micturition is lowered or lacked. Urinary incontinence includes urge urinary incontinence, stress urinary incontinence, overflow urinary incontinence and reflex urinary incontinence and a GABA uptake inhibitor is particularly effective for urge urinary incontinence.

Furthermore, urinary frequency and urinary incontinence include nocturnal enuresis of infants or senilities, and abnormal micturition accompanied with diseases such as dementia.

GABA uptake inhibitors, in particular, the aforementioned compounds A to O are low toxic and safe and are useful as a composition for preventing or treating or as a drug for improving abnormal micturition such as urinary frequency and urinary incontinence for a mammal (such as mouse, rat, hamster, rabbit, cat, dog, cattle, sheep, monkey and human being).

The preventing or treating composition of the present invention may be in the form of any of solid preparations such as powders, fine granules, granules, tablets and capsules, and liquid preparations such as syrups suspensions, emulsions and injectables.

The preventing or treating composition of the present invention can be prepared by the conventional method such as mixing, kneading, granulating, tabletting, coating, sterile treating and emulsifying preventing upon the form of preparations. Regarding production of a preparation, for example, respective items of Japanese Pharmacopoeia, general rules for preparations can be referenced.

The content of a GABA uptake inhibitor in the preventing or treating composition of the present invention is different depending upon the form of preparations and is usually about 0.01 to 100% by weight, preferably about 0.1 to 50% by weight, more preferably about 0.5 to 20% by weight relative to the whole preparation.

When a GABA uptake inhibitor is used for the aforementioned medicament, it can be administered orally or parenterally, as it is, or in the form of solid preparations such as powders, fine granules, granules, tablets, coating tablets and capsules, or liquid preparations such as syrups, suspensions, emulsions and injectables by mixing with a suitable pharmacologically acceptable carrier, for example, an excipient (such as starch, lactose, scrouse, calcium carbonate and calcium phophate), a binding agent (such as starch, gum arabic, carboxymethylcellulose, hydroxypropylcellulose, crystalline cellulose, alginic acid, gelatin and poly(vinyl pyrrolidone)), a lubricant (such as stearic acid, magnesium stearate, calcium stearate and talc), a disintegrating agent (such as calcium carboxymethylcellulose and talc), a diluent (such as water for injection, physiological saline and propylene glycol water) and, if necessary, an additive (such as stabilizer, preservative, colorant, perfume, solubilizer, emulsifier, buffer and isotonic) according to a conventional method.

A dose is different depending upon a kind of a GABA uptake inhibitor, route of administration, and conditions and an age of a patient and, when orally administered to an adult patient of urinary frequency, about 0.005 to 50 mg, preferably about 0.05 to 10 mg, more preferably about 0.2 to 4 mg of a GABA uptake inhibitor/kg weight/day can be administered once to thrice in a divided manner.

Also, a GABA uptake inhibitor can be conveniently used together with a suitable amount of other drugs for preventing or treating urinary frequency and urinary incontinence. As such the drug for preventing or treating urinary frequency and urinary incontinence, for example, compounds described in JP-A 9-263585 (such as (9R)-7-[3,5-bis(trifluoromethyl)benzyl)-6,7,8,9,10,11-hexahydro-9-methyl-5-(4-methylphenyl)-6,13-dioxo-13H-[1,4]diazosino[2,1-g][1,7]naphthylidine) or salts thereof are used.

An amount of these other drugs for preventing or treating urinary frequency and urinary incontinence to be used is usually about 0.01 to 90% by weight, preferably about 0.1 to 50% by weight, more preferably about 0.5 to 20% by weight relative to the whole preparation.

The present invention will be explained in more detail by way of the following Examples but the present invention is not limited by the following Examples and a variation is possible in a range without departing the scope of the present invention.

| Example 1 Coated tablet | | |
|---|---|---|
| (1) | NO-711 hydrochloride | 10.0 mg |
| (2) | Lactose | 60.0 mg |
| (3) | Corn starch | 35.0 mg |
| (4) | Hydroxypropylmethylcellulose | 3.0 mg |
| (5) | Magnesium stearate | 2.0 mg |

A mixture of 10.0 mg of NO-711 (compound A) hydrochloride, 60.0 mg of lactose and 35.0 mg of corn starch is granulated using 0.03 ml of a 10% by weight aqueous hydroxypropylmethylcellulose solution (3.0 mg as hydroxypropylmethylcellulose), dried at 40°C and passed through a sieve. The resulting granule is mixed with 2.0 mg of magnesium stearate, and compressed. The resulting crude tablet is coated with a sugar coating of an aqueous suspension of sucrose, titanium dioxide, talc and gum arabic. The coated tablet is polished with beewax to obtain the desired coated tablet.

| Example 2 Tablet | | |
|---|---|---|
| (1) | NO-711 hydrochloride | 10.0 mg |
| (2) | Lactose | 70.0 mg |
| (3) | Corn starch | 50.0 mg |
| (4) | Soluble starch | 7.0 mg |
| (5) | Magnesium stearate | 3.0 mg |

10.0 mg of NO-711 hydrochloride and 3.0 mg of magnesium stearate are granulated with 0.07 ml of an aqueous soluble starch solution (7.0 mg as soluble starch), dried, and mixed with 70.0 mg of lactose and 50.0 mg of corn starch. The mixture is compressed to obtain the desired tablet.

| Example 3 Capsule | | |
|---|---|---|
| (1) | NO-711 hydrochloride | 25 mg |
| (2) | Lactose | 55 mg |
| (3) | Talc | 16 mg |
| (4) | Magnesium stearate | 4 mg |

The ingredients of the above ratio are filled in a standard 2-piece hard gelatin capsule to obtain the desired capsule.

### Example 4 Injectable preparation

NO-711 hydrochloride is dissolved in 10% propylene glycol water at 1.5% by weight to obtain the desired injectable preparation.

| Example 5 Coated tablet | | |
|---|---|---|
| (1) | Tiagabin hydrochloride | 10.0 mg |
| (2) | Lactose | 60.0 mg |
| (3) | Corn starch | 35.0 mg |
| (4) | Hydroxypropylmethylcellulose | 3.0 mg |
| (5) | Magnesium stearate | 2.0 mg |

A mixture of 10.0 mg of tiagabin hydrochloride (hydrochloride of compound C-1), 60.0 mg of lactose and 35.0 mg of corn starch is granulated using 0.03 ml of a 10% by weight aqueous hydroxypropylmethylcellulose solution (3.0 mg as hydroxypropylmethylcellulose), dried at 40°C and passed through a sieve. The resulting granule is mixed with 2.0 mg of magnesium stearate, and compressed. The resulting crude tablet is coated with a sugar coating of an aqueous suspension containing sucrose, titanium oxide, talc and gum arabic. The coated tablet is polished with beewax to obtain the desired coating tablet.

| Example 6 Tablet | | |
|---|---|---|
| (1) | Tiagabin hydrochloride | 10.0 mg |
| (2) | Lactose | 70.0 mg |
| (3) | Corn starch | 50.0 mg |
| (4) | Soluble starch | 7.0 mg |
| (5) | Magnesium stearate | 3.0 mg |

10.0 mg of tiagabin hydrochloride and 3.0 mg of magnesium stearate are granulated with 0.07 ml of an aqueous soluble starch solution (7.0 mg as soluble starch), dried, and mixed with 70.0 mg of lactose and 50.0 mg of corn starch. The mixture is compressed to obtain the desired tablet.

| Example 7 Capsule | | |
|---|---|---|
| (1) | Tiagabin hydrochloride | 25 mg |
| (2) | Lactose | 55 mg |
| (3) | Talc | 16 mg |
| (4) | Magnesium stearate | 4 mg |

The ingredients of the above ratio are filled into a standard 2-piece hard gelatin capsule to obtain the desired capsule.

### Example 8 Injectable preparation

Tiagabin hydrochloride is dissolved in 10% propylene glycol water at 1.5% by weight to obtain the desired injectable.

### Example 9

According to the same manner as those in Examples 1 to 4, preparations are prepared by using SKF89976A hydrochloride (hydrochloride of compound B) and compounds D to O, respectively.

### Test Example 1

The urinary frequency and urinary incontinence suppressing activity of NO-711 hydrochloride was evaluated by the ability to increase an interval between rhythmic bladder contractions in urethane-anesthetized guinea pig as an index. In this model, an urethra was ligated, a constant volume of a physiological saline (3 to 4.5 ml) warmed at 39°C was injected into the bladder to distend, and the regular rhythmic bladder contractions at a constant interval were observed. After the stable rhythmic bladder contractions were confirmed, a compound dissolved in physiological saline was administered intravenously to investigate the activity. Immediately after administration of a drug, the time of complete suppression of the rhythmic bladder contractions (rhythmic bladder contraction shutdown time, maximum observation period was 30 minutes) was measured. The results are shown in Table 1.

**Table 1**

| Administered drug | Dose (mg/kg) | Number of aminals | Rhythmic bladder contraction shutdown time(minutes, mean± standard error) |
|---|---|---|---|
| Physiological saline | - | 7 | 3.5±1.3 |
| NO-711 hydrochloride | 1 | 6 | 5.9±1.3 |
| | 3 | 7 | 18.4±5.0* |
| | 10 | 7 | 16.9±3.6** |

A drug was administered intravenously.
*P<0.05, **P<0.01 (significance to the physiological saline-administered control group, nonparametric Dunnett multiple comparison test, two-tailed test)

### Test Example 2

The urinary frequency and urinary incontinence suppressing activity of a GABA uptake inhibitor was measured as the ability to abolish the rhythmic bladder contractions in urethane-anesthetized rats as an index. An urethra was ligated, a constant volume of physiological saline warmed at 39°C was injected into the bladder to distend the detrusor muscle, and the regular rhythmic bladder contractions at a constant interval were observed. After the stable bladder contractions were confirmed, a compound was administered intravenously to investigate the activities. A time for completely suppressing the rhythmic bladder contractions after the drug administration (reflex shutdown time, a maximum observation period was 30 minutes) and a maximum contractile intravesical pressure at which the bladder contraction appeared again after the administration was measured. The results are shown in Table 2. The GABA uptake inhibitor increased a reflex shutdown time without affecting the maximum contractile pressure.

**Table 2**

| Drug | Dose (mg/ kg) | Reflex shutdown time | | Change in maximum contractile intravesical pressure | |
|---|---|---|---|---|---|
| | | (Number of animals) | (min.) | (Number of animals) | (Relative to before administration, %) |
| | 0 | 6 | 4.1±0.7 | 6 | -5.9±3.4 |
| | 0.3 | 6 | 4.7±1.6 | 6 | -12.6±5.8 |
| Tiagabin hydrochloride | 1 | 6 | 11.6±3.8 | 5 | 4.5±17.3 |
| | 3 | 6 | 18.0±3.5* | 5 | -19.4±11.8 |
| | 10 | 5 | 24.6±3.1** | 4 | -31.6±10.5 |
| | 0 | 6 | 2.0±0.5 | 6 | -6.2±3.4 |
| NO-711 | 0.3 | 6 | 3.8±1.1 | 6 | -3.3±3.2 |
| hydrochloride | 1 | 6 | 11.4±2.9** | 6 | -19.7±5.6* |
| | 3 | 6 | 12.1±2.5** | 6 | -8.4±5.8 |
| | 0 | 6 | 3.5±0.8 | 6 | 11.7±7.1 |
| SKF89976A | 3 | 6 | 5.0±1.0 | 6 | -7.1±3.1 |
| hydrochloride | 10 | 6 | 15.7±3.3* | 6 | -12.1±11.5 |
| | 30 | 5 | 25.1±4.6** | 3 | -8.4±10.6 |

Tiagabin hydrochloride was dissolved in distilled water, other drugs were dissolved in physiological saline, and the drugs were administered intravenously, respectively. Data are shown as mean ± standard error.
*P<0.05, **P<0.01 (significance relative to the vehicle-administered control group, nonparametric Dunnett multiple comparison test, two-tailed test)
^{#}P<0.05 (significance to the values obtained before drug administration, paired t test, two-tailed test). A maximum contractile pressure was calculated from animals from which the bladder constriction was obtained in an observation period.

### Test Example 3

The urinary frequency and urinary incontinence suppressing activity of a GABA uptake inhibitor was shown by the ability to increase bladder capacity in urethane-anesthetized rats. After a bladder was made empty by drawing out the urine, physiological saline warmed at 39°C was injected into the bladder at a constant rate (0.05 ml/min.) to induce micturition. These procedures were repeated to confirm stable bladder capacity (volume of physiological saline injected until start of micturition) and, thereafter, compounds were administered intravenously to investigate the activities. A change in bladder capacity and micturition pressure after the administration was measured. The results are shown in Table 3. The GABA uptake inhibitor increased bladder capacity.

**Table 3**

| Drug | Dose (mg/ kg) | Change in bladder capacity | | Change in micturition pressure | |
|---|---|---|---|---|---|
| | | (Number of animals) | (Relative to before administration, %) | (Number of animals) | (Relative to before administration, %) |
| Distilled water | | 5 | -15.0±18.7 | 5 | -5.0±13.2 |
| | | | | | |
| Tiagabin hydrochloride | 1 | 7 | 42.7±35.6 | 7 | 15.2±14.6 |
| | | | | | |
| | 3 | 6 | 236.8±81.2* | 6 | 29.5±15.6 |
| | 10 | 6 | 276.4±29.4** | 6 | 2.5±10.3 |
| | | | | | |
| | 0.3 | 6 | -8.4±11.3 | 6 | -18.1±10.5 |
| NO-711 hydrochloride | 1 | 8 | 180.9±76.8* | 8 | 16.4±8.2 |
| | 3 | 8 | 188.7±45.3** | 8 | 14.6±18.2 |

Tiagabin hydrochloride was dissolved in distilled water, other drugs were dissolved in physiological saline and the drugs were administered intravenously, respectively. Data are shown as the mean ± standard error. After drug administration, animals showing an overflow urinary incontinence-like cystometrogram (urine leakage without the bladder constriction) were excluded from calculation of micturition pressure.
*P<0.05, **P<0.01 (significance to the values obtained before drug administration, paired t test, two-tailed test)

### Test Example 4

The urinary frequency and urinary incontinence suppressing activity of a GABA uptake inhibitor was measured as the ability to increase the micturition interval in bilateral common carotid artery-ligated rat under consciousness as an index. An animal was restricted in a Ballmann cage, and physiological saline was sustained-injected into the bladder at a constant rate (0.1 ml/min.) to induce repeated micturition. After the stable micturition interval was confirmed, common carotid arteries were ligated bilaterally and micturition interval was decreased. A compound was administered intravenously to animals with micturition interval decreased 20% or more following ligation, and the activity was investigated. The micturition interval and micturition pressure before and after drug administration were measured for 30 minutes, and the averages are expressed as the ratio to the value before bilateral common carotid artery-ligation (Table 4). A GABA uptake inhibitor, tiagabin hydrochloride, showed an increasing effect on micturition interval which had been decreased by bilateral common carotid artery ligation.

**Table 4**

| Drug | Dose (mg/ kg) | Number of animals | Micturition interval (relative to before ligation, %) | | Micturition pressure (relative to before ligation, %) | |
|---|---|---|---|---|---|---|
| | | | Before administration | After administration | Before administration | After administration |
| Distilled water | | 6 | 56.1±6.9 | 60.7±8.2 | 88.4±4.4 | 89.3±4.4 |
| | | | | | | |
| Tiagabin hydrochloride | 0.1 | 5 | 58.5±3.1 | 78.8±10.0 | 103.9±1.1 | 100.7±2.3 |
| | 0.3 | 6 | 62.5±4.9 | 92.1±12.8* | 91.3±10.6 | 86.3±9.2 |
| | 1 | 4 | 63.2±3.3 | 100.0±6.5** | 111.5±8.3 | 105.0±8.4 |
| | 3 | 4 | 46.5±11.6 | 75.5±15.0* | 105.2±10.8 | 106.2+5.3 |
| Physiological saline | | 4 | 56.2±3.6 | 69.2±10.3 | 103.9±5.9 | 99.6±7.8 |

Tiagabin hydrochloride was dissolved in distilled water and administered intravenously. Crystometograms for 30 minutes before and after drug administration were continuously measured and compared. Data are shown as a ratio to micturition interval and micturition pressure before bilateral common carotid artery-ligation (mean ± standard error).
*P<0.05, **P<0.01 (significance relative to before drug administration, paired t test, two-tailed test)

### Test Example 5

The urinary frequency and urinary incontinence activity of a GABA uptake inhibitor was measured as the ability to increase the micturition interval in a unilaterally decerebrated rat under consciousness as an index. Animals were restricted in a Ballmann cage, and physiological saline was sustained-injected into the bladder at a constant rate (0.05 ml/min.) to induce repeated micturition. In a unilaterally decerebrated rat, micturition interval was decreased, and after the stable micturition interval was confirmed, a drug was administered intravenously or orally to investigate the activity (Table 5 and Table 6). The micturition interval and micturition pressure before and after drug administration were measured for 30 minutes, and the mean values were compared.

**Table 5**

| Drug | Dose (mg/ kg) | Number of animals | Micturition interval (min.) | | Micturition pressure (mmHg) | |
|---|---|---|---|---|---|---|
| | | | Before administration | After administration | Before administration | After administration |
| Normal animal Unilaterally decerebrated animal | | 5 | 12.7±2.0 | | 15.3±1.7 | |
| Distilled water | | 5 | 5.5±1.4 | 5.3±1.0 | 13.9±1.4 | 14.8±1.6 |
| Tiagabin hydrochloride | 0.3 | 4 | 6.6±1.5 | 6.8±0.9 | 13.9±2.3 | 15.1±2.6 |
| | 1 | 5 | 5.2±0.7 | 6.5±1.0* | 14.4±1.4 | 14.5±1.5 |
| | 3 | 5 | 5.1±0.5 | 7.3±0.5** | 16.1±1.4 | 16.5±1.6 |

A drug was administered intravenously. Cystometogram was continuously measured for 30 minutes starting 15 minutes after drug administration, and it was compared with that for 30 minutes before drug administration. Data are shown as the means ± standard error.
*P<0.05 **P<0.01 (significance to before administration, paired t test, two-tailed test)

**Table 6**

| Drug | Dose (mg/ kg) | Number of specimens | Micturition interval (min.) | |
|---|---|---|---|---|
| | | | Before administration | After administration |
| Distilled water | | 7 | 5.1±0.6 | 5.1±0.4 |
| Tiagabin hydrochloride | 3 | 7 | 4.9±0.8 | 5.6±0.7 |
| | 10 | 7 | 4.4±0.5 | 6.1±0.8* |

A drug was administered orally. A bladder inner pressure curve was continuously measured for 30 minutes starting 60 minutes after drug administration, and it was compared with that for 30 minutes before drug administration. Data are shown as the mean ± standard error.
*P<0.05 (significance relative to drug administration, paired t test, two-tailed test)

As apparent in Table 5 and Table 6, tiagabin hydrochloride which is a GABA uptake inhibitor increased micturition interval in a unilaterally decerebrated rat in both intravenous and oral administrations.

### Industrial Applicability

A GABA uptake inhibitor is useful as a composition for preventing or treating urinary frequency and urinary incontinence.

## Claims

1. A composition for preventing or treating urinary frequency and urinary incontinence, which comprises a GABA uptake inhibitor.

2. The composition according to claim 1, wherein the GABA uptake inhibitor is;
1) 1-(2-((diphenylmethylene)amino)oxy)ethyl)-1,2,5,6-tetrahydro-3-pyridinecarboxylic acid represented by the formula: 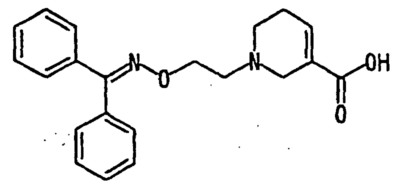 or a salt thereof,
2) (R)-1-(4,4-diphenyl-3-butenyl)-3-piperidinecarboxylic acid represented by the formula: 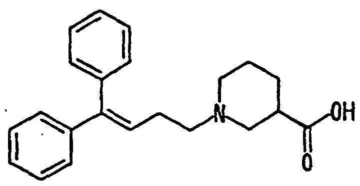 or a salt thereof,
3) a compound represented by the formula: 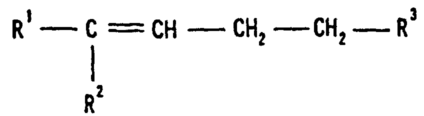 wherein R¹ and R² are the same or different, and each represents furanyl, thienyl, pyridyl or pyrrolyl, which may be substituted with 1 to 3 halogen or lower alkyl, and R³ represents 3-carboxypiperidin-1-yl, 3-carboxy-1,2,5,6-tetrahydropyrid-1-yl or 3-carboxymethylpyrrolidin-1-yl, or a salt thereof,
4) 1,2,5,6-tetrahydronicotinic acid represented by the formula: 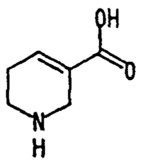 or a salt thereof,
5) N-(4,4-diphenyl-3-butenyl)-3-pyrroridineacetic acid or a salt thereof,
6) 3-piperidinecarboxylic acid or a salt thereof,
7) 1,2,5,6-tetrahydro-3-pyridinecarboxylic acid or a salt thereof,
8) N-(4,4-diphenyl-3-butenyl)-1,2,5,6-tetrahydro-3-pyridinecarboxylic acid or a salt thereof,
9) cis-4-hydroxyl-3-piperidinecarboxylic acid or a salt thereof,
10) 6-(trifluoromethoxy)-2-benzothiazole-amine represented by the formula: 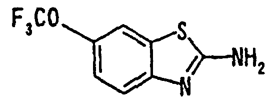 or a salt thereof, or
11) a compound represented by the formula: 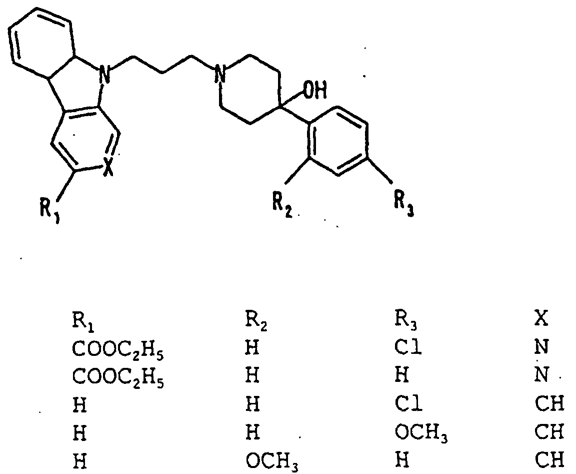 or a salt thereof.

3. The composition according to claim 1, which is a composition for preventing or treating urinary frequency.

4. The composition according to claim 3, wherein the GABA uptake inhibitor is:
1) 1-(2-((diphenylmethylene)amino)oxy)ethyl)-1,2,5,6-tetrahydro-3-pyridinecarboxylic acid represented by the formula: 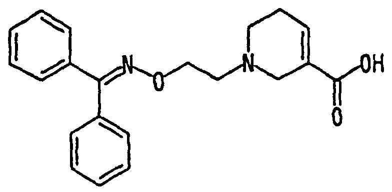 or a hydrochloride thereof,
2) (R)-1-(4,4-diphenyl-3-butenyl)-3-piperidinecarboxylic acid represented by the formula: 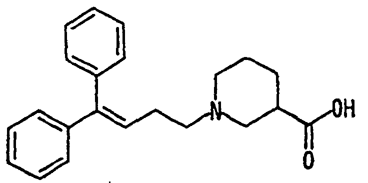 or a hydrochloride thereof, or
3) (R)-1-[4,4-bis(3-methyl-2-thienyl)-3-butenyl]-3-piperidinecarboxylic acid represented by the formula: 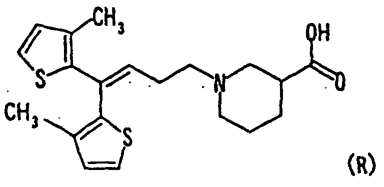 or a hydrochloride thereof.

5. The composition according to claim 1, which is a composition for preventing or treating urinary incontinence.

6. A composition for preventing or treating urinary incontinence, which comprises containing a GABA uptake inhibitor containing no sulfur atom.

7. The composition according to claim 5 or 6, wherein the GABA uptake inhibitor is:
1) 1-(2-((diphenylmethylene)amino)oxy)ethyl)-1,2,5,6-tetrahydro-3-pyridinecarboxylic acid represented by the formula: 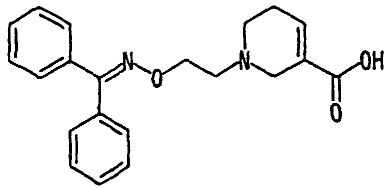 or a salt thereof,
2) (R)-1-(4,4-diphenyl-3-butenyl)-3-piperidinecarboxylic acid represented by the formula: 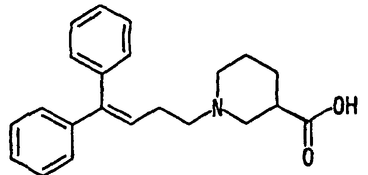 or a salt thereof,
3) 1,2,5,6-tetrahydronicotinic acid represented by the formula: 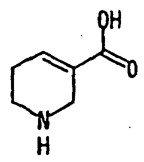 or a salt thereof,
4) N-(4,4-diphenyl-3-butenyl)-3-pyrrolidineacetic acid or a salt thereof,
5) 3-piperidinecarboxylic acid or a salt thereof,
6) 1,2,5,6-tetrahydro-3-pyridinecarboxylic acid or a salt thereof,
7) N-(4,4-diphenyl-3-butenyl)-1,2,5,6-tetrahydro-3-pyridinecarboxylic acid or a salt thereof,
8) cis-4-hydroxy-3-piperidinecarboxylic acid or a salt thereof,
9) 6-(trifluoromethoxy)-2-benzothiazole-amine represented by the formula: 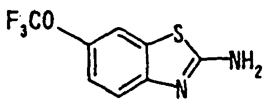 or a salt thereof, or
10) a compound represented by the formula: 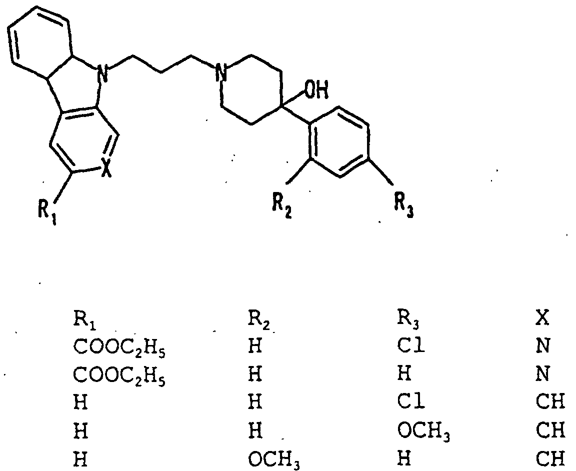 or a salt thereof.

8. The composition according to claim 5 or 6, wherein the GABA uptake inhibitor is:
1) 1-(2-((diphenylmethylene)amino)oxy)ethyl)-1,2,5,6-tetrahydro-3-pyridinecarboxylic acid represented by the formula: 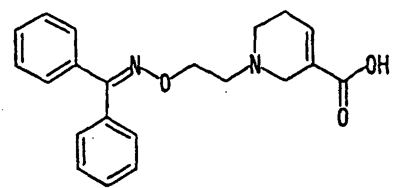 or a hydrochloride thereof, or
2) (R)-1-(4,4-diphenyl-3-butenyl)-3-piperidinecarboxylic acid represented by the formula: 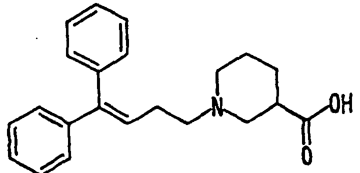 or a hydrochloride thereof.

9. The composition according to claim 5, wherein the GABA uptake inhibitor is a compound represented by the formula: 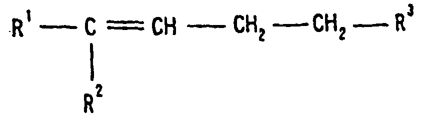 wherein R¹ and R² are the same or different, and each represents furanyl, thienyl, pyridyl or pyrrolyl, which may be substituted with 1 to 3 halogen or lower alkyl, and R³ represents 3-carboxy piperidin-1-yl, 3-carboxy-1,2,5,6-tetrahydropyrid-1-yl or 3-carboxymethylpyrrolidin-1-yl, or a salt thereof.

10. The composition according to claim 5, the GABA uptake inhibitor is (R)-1-[4,4-bis(3-methyl-2-thienyl)-3-butenyl]-3-piperidinecarboxylic acid represented by the formula: 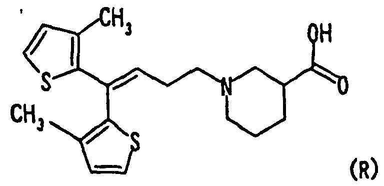 or a hydrochloride thereof.

11. The composition according to any one of claims 5 to 10, wherein urinary incontinence is urgency urinary incontinence.

12. A method for preventing or treating urinary frequency and urinary incontinence, which comprises administering an effective amount of a GABA uptake inhibitor to a mammal.

13. Use of a GABA uptake inhibitor for preparing an agent for preventing or treating urinary frequency and urinary incontinence.
